# EUROPÄISCHE PATENTSCHRIFT

(11) **EP 1 295 565 B1**
(45) Veröffentlichungstag und Bekanntmachung des Hinweises auf die Patenterteilung: **28.01.2004**
(21) Anmeldenummer: 01122415.1
(22) Anmeldetag: 20.09.2001
(51) Int. Cl.: A61B 17/16, A61B 17/92

(54) **Medizinisches Werkzeug**
Medical instrument
Instrument médical

(43) Veröffentlichungstag der Anmeldung: 26.03.2003
(73) Patentinhaber: Karl Storz GmbH & Co. KG, 78532 Tuttlingen (DE)
(72) Erfinder: Wolff, Wilhelm, Dr. med., 6370 Kitzbühel (AT); Klein, Dov, Dr. med., Savion 56512 (IL)
(74) Vertreter: Hofmeister, Frank Horst

(56) Entgegenhaltungen:
- EP-A- 0 780 090
- US-A- 4 697 586
- US-B1- 6 174 311

## Beschreibung

Die Erfindung betrifft ein medizinisches Werkzeug zum Durchtrennen, Abtragen und insbesondere Modellieren von Knochen, Knorpeln und dergleichen, insbesondere ein Nasenrückenosteotom, umfassend einen Meißel mit einem Schaft und einer am distalen Ende des Schaftes ausgebildeten Meißelschneide.

Die Osteotomie ist eine operative Methode zum Durchtrennen von Knochen und dergleichen mittels eines Meißels oder einer Säge, um beispielsweise Fehlstellungen zu beseitigen.

Dokument US-A-4,697,586 offenbart ein medizinisches Werkzeug nach dem Oberbegriff des Anspruchs 1.

Für die Osteotomie im Nasenrückenbereich werden bei den herkömmlichen Operationstechniken für den jeweiligen Verwendungszweck speziell ausgeformte Meißel verwendet, die mittels eines Hammers vorangetrieben werden. Hierzu führt der Operateur mit einer Hand den Meißel und schlägt mit dem mit der anderen Hand gegriffenen Hammer auf das der Meißelschneide entgegengesetzte proximale Ende des Meißelschaftes. Nachteilig bei dieser bekannten Operationstechnik ist, daß auch bei Anwendungen, wie beispielsweise dem Modellieren eines Nasenrückens, bei denen eine äußerst präzise Führung des Meißel notwendig ist, der Meißel direkt mit der Hand des Operateurs geführt wird. Beim Einschlagen des Meißels mittels des Hammers kann es leicht passieren, daß die den Meißel führende Hand ein wenig verlagert wird, was zu einer nicht optimalen Bearbeitung des jeweiligen Knochens führen kann.

Davon ausgehend liegt der Erfindung die **Aufgabe** zugrunde, ein medizinisches Werkzeug der eingangs genannten Art bereitzustellen, das eine einfache und präzise Führung des Meißels ermöglicht.

Die **Lösung** dieser Aufgabenstellung ist erfindungsgemäß gekennzeichnet durch eine Handhabe, die einen Handgriff sowie eine Führung zur Aufnahme des Meißels umfaßt, wobei der Handgriff und die Führung räumlich voneinander beabstandet sind.

Durch die Verwendung der den Meißel aufnehmenden Handhabe besteht erstmalig die Möglichkeit, den Meißel unabhängig von einer Lagerung direkt in der Hand insbesondere beim Hämmern präzise zu führen. Die Lagerung des Meißels an der Handhabe ermöglicht eine einfachere und sichere Aufnahme der Hammerschläge, ohne die Gefahr, daß bei jedem Hammerschlag die den Meißel führende Hand aus der für den jeweiligen Abtragvorgang notwendigen Position verrutscht.

Über den Handgriff läßt sich der in die Führung eingesetzte Meißel einfach und präzise in die gewünschte Arbeitsstellung bringen und auch während des Hämmerns in von dieser Position ausgehenden vorbestimmten Bahnen führen.

Um eine spielfreie und kippstabile Lagerung des Meißels in der Führung der Handhabe zu gewährleisten, wird gemäß einer ersten Ausführungsform der Erfindung vorgeschlagen, daß die Führung als in einem Abschnitt der Handhabe angeordneter Schlitz ausgebildet ist, wobei die Handhabe in diesem Abschnitt den Querschnitt eines Abschnitts des Meißels zumindest teilweise umgreift.

Gemäß einer zweiten Ausführungsform der Erfindung ist die Führung als an der Handhabe angeordnete Führungsvorrichtung ausgebildet ist, die den Querschnitt eines Abschnitts des Meißels zumindest teilweise umgreift, so daß der Meißel in der Führungsvorrichtung geführt ist. Insbesondere bei dieser Ausgestaltungsform ist es möglich, den Meißel nur seitlich und bezüglich einer Drehung um die Meißellängsachse zu führen, dahingegen aber ein Kippen um die Meißelquerachse zuzulassen.

Damit der Meißel bei der Hämmerbewegung spielfrei und genau geführt werden kann, entspricht die Einstecktiefe des Meißels in die Führung vorzugsweise einem Vielfachen der Höhe des Querschnitts des Meißels.

Für zumindest die Teile des Meißelschaftes sowie die Führung, die die Gleitpaarung zum Verschieben des Meißels bilden, sind vorzugsweise Materialien mit guten Gleiteigenschaften, insbesondere einem niedrigen Reibungskoeffizienten, auszuwählen, um auch bei ungünstiger Lagerung und ungünstigen Arbeitsbedingungen ein dauerhaft sicheres Gleiten ohne jegliche Spanabhebung sicherzustellen.

Damit auch die unterschiedlichsten Meißel, wie beispielsweise solche mit einem geraden, gewölbten oder gekanteten Schaft präzise in der Führung lagerbar sind, entspricht die Führung im Längs- und Querschnitt der Längs- und Querschnittsform des in der Führung gelagerten Abschnitts des Meißels.

Weiterhin wird mit der Erfindung vorgeschlagen, daß zur Begrenzung der Einschlaglänge des Meißels an der Führung und/oder am Meißel ein Anschlag ausgebildet ist, so daß der Meißel exakt nur bis zum vorgegebenen Zielpunkt eingeschlagen werden kann. Der Anschlag kann dabei so ausgebildet sein, daß er das Einschlagen des Meißels fest oder auch dämpfend begrenzt.

Bei der Ausbildung des Anschlags am Meißel ist der Anschlag vorzugsweise als im Bereich der Führung gegen die Handhabe oder Führungsvorrichtung anlaufende Vergrößerung des Querschnitts des Schaftes des Meißels ausgebildet, wobei dieser Querschnittssprung in der Meißelhauptebene und/oder in der zu dieser Ebene senkrechten Ebene durch die Meißelmittelachse ausgebildet sein kann und einen Anschlag ohne Klemmwirkung bildet.

Gemäß einer bevorzugten Ausführungsform der Erfindung, insbesondere für die offene Operationsmethode, ist der Meißel über ein auf den Schaft des Meißels aufschiebbares Gewicht in Arbeitsrichtung antreibbar, wobei am Schaft des Meißels ein Anschlag als Widerlager für das Gewicht ausgebildet ist. Das Einschlagen des Meißels über das als Gleithammer wirkende, auf den Meißelschaft aufgesetzte Gewicht gewährleistet, daß die über den Hammer bzw. das Gewicht auf den Meißel aufgebrachte Kraft immer genau gerade in Meißelrichtung auf den Meißel wirkt. Ein schräges Auftreffen oder sogar ein Abrutschen des Hammers ist bei dieser Ausgestaltungsform konstruktionsbedingt ausgeschlossen. Darüber hinaus ist ein solchermaßen ausgestaltetes Werkzeug auch unter eingeschränkten Platzverhältnissen, bei denen die Verwendung eines separaten Hammers kaum möglich ist, sicher einsetzbar.

Das als Gleithammer entlang dem Meißelschaft verschiebbare Gewicht kann aus verschiedenen Materialien und/oder mehreren Teilen aus unterschiedlichen Werkstoffen bestehen. Vorzugsweise werden körperverträgliche Materialien, wie beispielsweise körperverträgliche Stahllegierungen verwendet, die teilweise auch kuststoffummantelt sein können.

Bei der Materialauswahl für zumindest die Teile des als Gleithammer wirkenden Gewichts sowie des Meißelschaftes, die die Gleitpaarung zum Verschieben des Gewichtes entlang dem Meißelschaft bilden, werden Materialien mit guten Gleiteigenschaften, insbesondere einem niedrigen Reibungskoeffizienten, bevorzugt, um auch bei ungünstiger Lagerung und ungünstigen Arbeitsbedingungen ein dauerhaft sicheres Gleiten ohne jegliche Spanabhebung sicherzustellen.

Durch eine ergonomisch optimierte Ausgestaltung der Außenkontur des Gewichts kann die Handhabung für den Operateur deutlich verbessert und erleichtert werden. Diese ergonomische Ausgestaltung kann beispielsweise in der Ausarbeitung von Greifflächen bestehen, wobei unterschiedliche Finger- und Handgrößen und - formen sowie Formen für Rechts- und Linkshänder möglich sind. Neben dem Ausarbeiten der ergonomischen Außenkontur durch Ein- oder Ausarbeiten von beispielsweise Greifflächen kann diese ergonomische Ausgestaltung auch dadurch erzielt werden, daß ergonomisch optimiert ausgestaltete Bauteile an dem Gewicht festlegbar sind, deren Material sich von dem des eigentlichen Gewichts unterscheiden kann.

Um bei der für die offene Operationsmethode bevorzugten Ausführungsform des erfindungsgemäßen Werkzeugs den konstruktiven Aufwand zur Ausgestaltung des Meißels möglichst klein zu halten, wird weiterhin vorgeschlagen, daß der das Widerlager für das Gewicht bildende Anschlag gleichzeitig auch den die Einschlaglänge des Meißels begrenzenden Anschlag bildet.

Zum Schutz der Haut im Operationsgebiet wird erfindungsgemäß vorgeschlagen, daß am Meißel im Bereich der Meißelschneide zumindest einseitig, vorzugsweise beidseitig, ein Meißelklingenschutz angeordnet ist, so daß die Meißelschneide nur in der Einschlagrichtung wirksam ist.

Bei der Materialauswahl für den Meißelklingenschutz, der aus verschiedenen Werkstoffen gefertigt sein kann, ist insbesondere darauf zu achten, daß Werkstoffe verwendet werden, die auf der Außenseite eine Verletzungsgefahr für den Operierenden sowie die Haut des Patienten ausschließen und auf der Innenseite bei einer eventuellen Berührung mit dem Meißel infolge einer versehentlichen Stoßoder Schlagbelastung eine Beschädigung des Meißels oder Meißelklingenschutzes, insbesondere eine Spanabhebung an einem der Teile, ausschließen.

Weiterhin wird mit der Erfindung vorgeschlagen, daß an der Handhabe ein als Retraktor wirkender Weichteilschutz angeordnet ist, um einerseits den Zugang zum Operationsgebiet zu erleichtern und andererseits umliegendes Gewebe zu schützen. Der Weichteilschutz kann dabei einstückig mit dem Meißelklingenschutz ausgebildet sein, jedoch ist es auch möglich, den Weichteilschutz als separat an der Handhabe festlegbares Bauteil auszubilden.

Das präzise Führen des Meißels kann erfindungsgemäß dadurch erleichtert werden, daß an der Handhabe eine den Zielpunkt des Meißels markierende Anlagevorrichtung festlegbar ist, die vorzugsweise einen Einschlagdorn zum Festlegen am Zielpunkt aufweist, so daß über die Anlagevorrichtung oder den Einschlagdorn die Handhabe am zu operierenden Körperteil fixiert ist.

Gemäß einer praktischen Ausführungsform der Erfindung bildet die den Zielpunkt des Meißels markierende Anlagevorrichtung gleichzeitig auch den als Retraktor wirkenden Weichteilschutz.

Weiterhin wird mit der Erfindung vorgeschlagen, daß die Anlagevorrichtung eine Hammerplattform zum Einschlagen des Einschlagdorns mit einem vom Meißel separaten Hammer aufweist.

Schließlich wird mit der Erfindung vorgeschlagen, daß zumindest alle direkt mit dem Patientenkörper, insbesondere Knochen- und/oder Knorpelteilen, in Kontakt kommenden Bauteile aus körperverträglichen Materialien, insbesondere köperverträglichen Stahllegierungen, hergestellt sind. Zu den Teilen des Werkzeugs, die insbesondere aus körperverträglichen Materialien herzustellen sind, zählen insbesondere der Meißel oder zumindesz die Meißelschneide, der Weichteilschutz sowie die Anlagevorrichtung und der Einschlagdorn, da diese Bauteile nicht nur direkt mit der Haut des Patienten in Kontakt kommen, sondern bei der offenen Nasenrükkenchirurgie sogar mit offengelegtem subkutanem Gewebe sowie teilweise Knochen- und Knorpelpartien in Kontakt kommen.

Weitere Merkmale und Vorteile der Erfindung ergeben sich anhand der Beschreibung der zugehörigen Zeichnung, in der zwei Ausführungsbeispiele eines erfindungsgemäßen medizinischen Werkzeugs nur beispielhaft schematisch dargestellt sind. In der Zeichnung zeigt:
- Fig. 1: eine Seitenansicht einer ersten Ausführungsform eines erfindungsgemäßen medizinischen Werkzeugs, vor dem Zusammenfügen der Einzelteile;
- Fig. 2a: eine Seitenansicht des Werkzeugs gemäß Fig. 1 im zusammengesetzten Zustand, eine erste Arbeitsposition darstellend;
- Fig. 2b: eine Fig. 2a entsprechende Ansicht, jedoch eine zweite Arbeitsposition darstellend;
- Fig. 3: eine Vorderansicht der Handhabe gemäß Fig. 1;
- Fig. 4: eine Vorderansicht des Meißels gemäß Fig. 1;
- Fig. 5: eine Vorderansicht des Gewichts gemäß Fig.1;
- Fig. 6: eine Seitenansicht einer zweiten Ausführungsform eines erfindungsgemäßen medizinischen Werkzeugs, vor dem Zusammenfügen der Einzelteile;
- Fig. 7: eine Draufsicht auf die Handhabe gemäß Fig. 6 und
- Fig. 8: eine Draufsicht auf den Meißel gemäß Fig. 6.

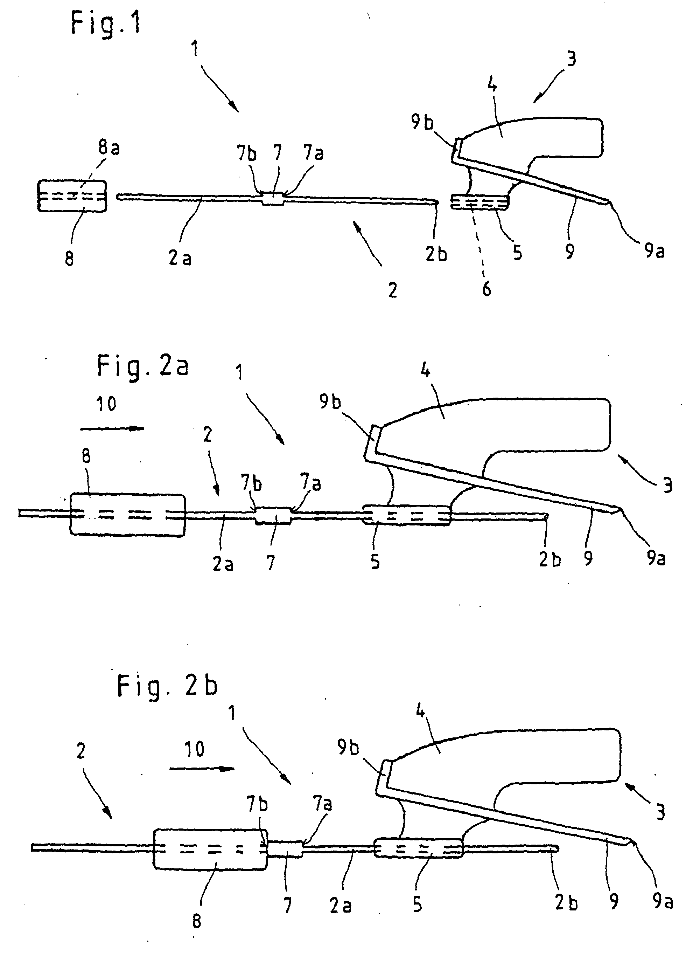

Bei den in den Abbildungen Fig. 1, 2a, 2b und 6 dargestellten medizinischen Werkzeugen 1 handelt es sich um Nasenrückenosteotome, wie diese beispielsweise zum Beseitigen von Knochenfehlstellungen im Nasenrückenbereich und zum Modellieren von Nasenrücken verwendet werden.

Diese medizinischen Werkzeuge 1 bestehen im wesentlichen aus einem Meißel 2 und einer Handhabe 3 zum Halten und Führen des Meißels 2. Die Handhabe 3 besteht bei beiden dargestellten Ausführungsformen jeweils aus einem Handgriff 4 und einer fest mit dem Handgriff 4 verbundenen Führung 5 zur Aufnahme des Meißels 2.

Der Meißel 2 oder zumindest die Abschnitte des Meißels 2, die in direkten Kontakt mit dem Patientenkörper kommen, das heißt insbesondere die Meißelschneide 2b, sind vorzugsweise aus einem körperverträglichen Material, beispielsweise einer körperverträglichen Stahllegierung ausgebildet.

Wie insbesondere aus den Abbildungen Fig. 1 bis 2b sowie Fig. 8 ersichtlich, besteht der Meißel 2 aus einem Schaft 2a, an dessen distalem Ende eine das eigentliche Werkzeug bildende Meißelschneide 2b ausgebildet ist. Im Gegensatz zu den dargestellten Meißeln 2 mit geradem Schaft 2a und rechteckigem Querschnitt existieren je nach Einsatzzweck auch Meißel 2 mit einem gewölbten oder gekanteten Längs- und/oder Querschnitt, die alle zum Halten und Führen an einer Handhabe 3 festlegbar sind, wobei die den jeweiligen Meißel 2 aufnehmende Führung 5 an den Längs- und Querschnitt des jeweiligen Abschnitts des Schaftes 2a des Meißels 2 angepaßt ist, der in der Führung 5 gelagert ist. Für eine lagegenaue Führung des Meißels 2 ist es somit ausreichend, daß der Längs- und Querschnitt des Schaftes 2a des Meißels 2 in dem Bereich der Gleitfürung in der Führung 5 der Längs- und Querschnittsform der Führung 5 entspricht. In den anderen Bereichen kann die Längs- und Querschnittsform des Schaftes 2a des Meißels 2 von der der Führung 5 abweichen.

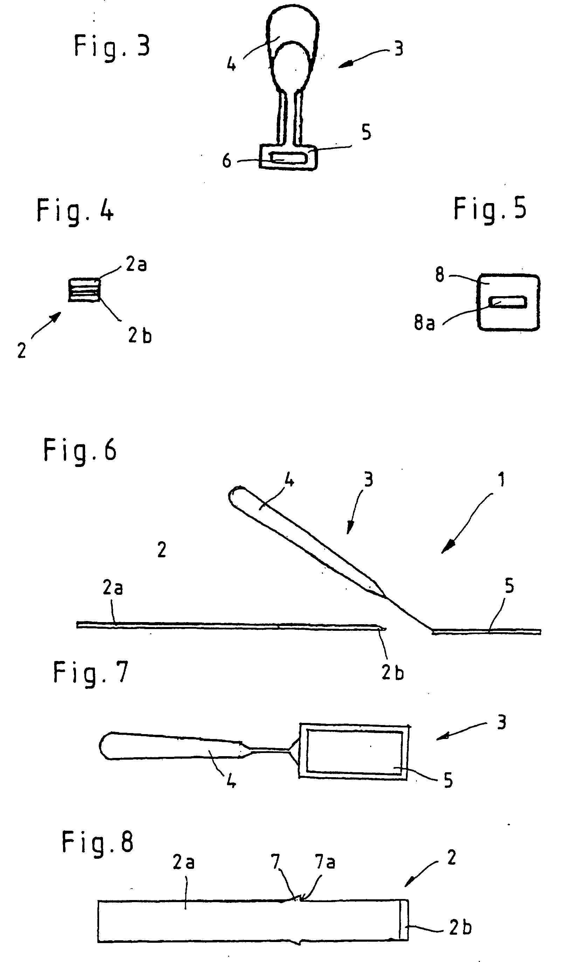

Während die in den Abbildungen Fig. 1 bis 5 gezeigte erste Ausführungsform bei der sogenannten offenen Methode zum Einsatz kommt, bei der das Operationsgebiet zuvor chirurgisch frei präpariert wird, findet das in den Abbildungen Fig. 6 bis 8 dargestellte Nasenrückenosteotom, bei der sogenannten geschlossenen Operationsmethode Verwendung, bei der der Zugang zum Operationsgebiet durch ein Nasenloch erfolgt.

Um den Meißel 2 lagegenau und kippstabil halten und führen zu können, ist bei der in den Abbildungen Fig. 1 bis Fig. 3 dargestellten ersten Ausführungsform die Führung 5 als in einem Abschnitt der Handhabe 3 angeordneter Schlitz 6 ausgebildet, wobei die Handhabe 3 in diesem Abschnitt den Querschnitt des Meißels 2 zumindest teilweise umgreift und die Einstecktiefe für den Schaft 2a des Meißels 2 einem Vielfachen der Höhe des Schaftquerschnitts entspricht. Wie aus Fig. 3 ersichtlich, weist bei dieser dargestellten Ausführungsform die Führung 5 einen rundum geschlossenen Schlitz 6 auf.

Bei der in den Abbildungen Fig. 6 und 7 dargestellten zweiten Ausführungsform ist Führung 5 als an der Handhabe 3 angeordnete Führungsvorrichtung ausgebildet ist, die den Querschnitt eines Abschnitts des Meißels 2 zumindest teilweise umgreift, so daß der Meißel 2 in der Führungsvorrichtung geführt ist. Bei dieser für die geschlossene Methode bevorzugten Ausführungsform umgreift die Führung 5 der Führungsvorrichtung den Meißel 2, führt ihn jedoch nur seitlich und bezüglich einer Drehung um die Meißellängsachse. Ein Kippen des Meißels 2 um die Meißelquerachse und eine geringe Bewegung des Meißels 2 nach oben und unten werden hierbei zugelassen.

Alternativ zu der dargestellten Ausführungsform kann die Führungsvorrichtung auch mehrteilig und eventuell zerlegbar so ausgebildet sein, daß sie nicht eine den Meißelschaft vollständig umschließende Führung bildet, sondern lediglich eine seitliche Verdrehsicherung darstellt, die zusätzlich obere und untere führende und stützende Gleitführungselemente umfaßt.

Zur Begrenzung der Einschlaglänge des Meißels 2 ist bei den abgebildeten Ausführungsformen am Schaft 2a des Meißels 2 ein den Querschnitt des Schaftes 2a vergrößernder Anschlag 7 ausgebildet, der beim Vorantreiben des Meißels 2 mit einer Anschlagfläche 7a gegen die Führung 5 der Handhabe 3 anläuft und so den Einschlagweg des Meißels 2 begrenzt. Bei den dargestellten Ausführungsbeispielen ist die Querschnittsvergrößerung durch eine Verbreiterung des Meißels 2 in der Meißelhauptebene ausgebildet. Alternativ oder zusätzlich kann diese Querschnittsvergrößerung aber auch in der zu dieser Ebene senkrechten Ebene ausgebildet sein. Für verschiedene Einschlaglängen sind dann unterschiedliche Meißel 2 mit entsprechend angeordneten Anschlägen 7 vorgesehen.

Ebenso ist es möglich, den Anschlag 7 zur Begrenzung der Einschlaglänge als am Meißel 2 verstellbar auszubilden. Hierzu kann der Anschlag 7 beispielsweise einen Schlitz aufweisen, der dem Schaft 2a des Meißels 2 angepaßt ist, um darauf zu gleiten. Der Anschlag 7 kann dann auf den Schaft 2a des Meißels 2 aufgeschoben werden und an einer oder verschiedenen dem Abtragungs- oder Operationszweck angepaßten Positionen beispielsweise durch Fixierung mittels einer Schraube in einer Nut festgelegt sein.

Neben der Ausbildung des die Einschlaglänge des Meißels 2 begrenzenden Anschlags 7 am Schaft 2a ist es selbstverständlich auch möglich, an der Führung 5 einen mit dem Schaft 2a zusammenwirkenden Anschlag auszubilden.

Das Antreiben des Meißels 2 in Arbeitsrichtung erfolgt über einen Hammer. Bei der in den Abbildungen Fig. 1 bis Fig. 5 dargestellten Ausführungsformen ist der für den Vortrieb des Meißels 2 dienende Hammer als auf den Schaft 2a des Meißels 2 aufsetzbares und entlang dem Schaft 2a verschiebbares Gewicht 8 ausgebildet. Wie insbesondere aus Fig. 5 ersichtlich, ist zum Verschieben des Gewichts 8 entlang dem Schaft 2a im Gewicht 8 ein Führungsschlitz 8a ausgebildet. Die Übertragung der Hammerkraft dieses als Gleithammer wirkenden Gewichts 8 auf den Meißel 2 erfolgt über einen am Schaft 2a des Meißels 2 ausgebildeten Anschlag 7. Wie in den Abbildungen Fig. 2a und 2b dargestellt, läuft das Gewicht beim Verschieben aus der Position gemäß Fig. 2a in die Position gemäß Fig. 2b gegen eine Anschlagfläche 7b des Anschlags 7 an und überträgt so die Schlagkraft auf den Meißel 2. Neben der dargestellten Ausführungsform, bei der der Anschlag 7 zur Begrenzung der Einschlaglänge des Meißels 2 und der das Widerlager für den Gleithammer dienende Anschlag 7 zusammenfallen, ist es auch möglich, zwei separate Anschläge am Schaft 2a des Meißels 2 auszubilden.

Bei der in den Abbildungen Fig. 6 bis 8 dargestellten zweiten Ausführungsform wird der Meißel 2 über einen separaten Hammer vorangetrieben, mit dem der Operateur auf das der Meißelschneide 2b entgegengesetzte Ende des Schaftes 2a schlägt. Selbstverständlich ist es auch bei diesem medizinischen Werkzeug 1 möglich, den separaten Hammer durch einen als verschiebbares Gewicht 8 ausgebildeten Gleithammer zu ersetzen, wie dies voranstehend zur ersten Ausführungsform des medizinischen Werkzeugs 1 beschrieben wurde.

Zum zielgenauen Festlegen der den Meißel 2 tragenden Handhabe 3 im Bereich des Operationsgebiets weist die Handhabe 3 eine Anlagevorrichtung 9 auf, deren distales Ende punktgenau auf den Zielpunkt des Meißels 2 einstellbar ist. Über einen am distalen Ende der Anlagevorrichtung 9 angeordneten Einschlagdorn 9a kann die Anlagevorrichtung 9 verrutschsicher fixiert werden, wobei am proximalen Ende derAnlagevorrichtung 9 eine Hammerplattform 9b ausgebildet ist, über die der Einschlagdorn 9a mit einem vom Meißel 2 separaten Hammer im Operationsgebiet festlegbar ist. Das Eindringen des Einschlagdorns 9a in die Knochen- oder Knorpelsubstanz macht eine körperverträgliche Werkstoffwahl für den Einschlagdorn 9a notwendig.

Bei der dargestellten Ausführungsform dient die Anlagevorrichtung 9 gleichzeitig auch als Weichteilschutz 9, der in der Art eines Retraktors nicht zu behandelndes Gewebe aus dem Operationsgebiet drängt. Vorteilhaft ist bei dieser Ausgestaltung, daß der Weichteilschutz 9 durch das Festlegen über den Einschlagdorn 9a der Anlagevorrichtung 9 beim Meißeln nicht verrutschen kann und der Zielpunkt für den Meißel 2 exakt definiert ist. Auch für den hier einstückig mit der Anlagevorrichtung 9 ausgeführten Weichteilschutz 9 ist wesentlich, daß dieser, der mit offengelegtem subkutanem Gewebe bzw. den inneren Hautschichten in Berührung kommt, aus körperverträglichem Material besteht.

Als weiterer Schutz für den Patienten ist es möglich, im Bereich der Meißelschneide 2b zumindest einseitig, vorzugsweise aber beidseitig, einen nicht dargestellten Meißelklingenschutz anzuordnen, um die Haut des Patienten ebenso wie den Operierenden vor versehentlichen Verletzungen durch die Meißelschneide 2b zu schützen. Der Meißelklingenschutz kann durch besonderes Ausformen der Anlagevorrichtung 9 ausgebildet sein oder an der Anlagevorrichtung 9 oder an einem anderen Abschnitt der Handhabe 3 angeordnet sein.

Die Verwendung des medizinischen Werkzeugs 1 gemäß Fig. 1 bis 6 geschieht wie folgt:

Ausgehend von der Abbildung Fig. 1 wird zunächst die Anlagevorrichtung 9 zielgenau an dem zuvor frei präparierten Nasenrücken des Patienten dadurch fixiert, daß der Einschlagdorn 9a mittels eines von dem Meißel 2 separaten Hammer über die Hammerplattform 9b in den Nasenrücken eingeschlagen wird, so daß der Zielpunkt des Meißels 2 exakt festgelegt ist. Anschließend wird der Meißel 2 in den Schlitz 6 der Führung 5 eingesetzt.

Zum Einschlagen des Meißels 2 wird nun das als Gleithammer wirkende Gewicht 8 auf den Schaft 2a des Meißels 2 aufgeschoben und ausgehend von der Position gemäß Fig. 2a in Richtung des Pfeils 10 bewegt, bis, wie in Fig. 2b dargestellt, das Gewicht 8 gegen die Anschlagfläche 7b des Anschlags 7 anläuft und so die Schlagkraft des Gewichtes 8 auf den Meißel 2 überträgt. Das Vorantreiben des Meißels 2 wird so lange fortgeführt, bis der Meißel 2 mit der Anschlagfläche 7a des Anschlags 7 gegen die Führung 5 anläuft, wodurch die Einschlaglänge des Meißels 2 begrenzt wird. Jetzt kann der Einschlagdorn 9a wieder aus dem Nasenrükken herausgezogen und das medizinische Werkzeug 1 entfernt werden. Gegebenenfalls kann der Anschlag 7 mit der Anschlagfläche 7a auch neu am Schaft 2a des Meißels 2 positioniert werden, beispielsweise indem eine in einer Nut fixierte Fixierungsschraube gelöst und in einer anderen Nut wieder eingeschraubt wird, so daß ein neuer Anschlag realisiert wird, der ein weiteres Abtragen des Nasenrükkens erlaubt.

Die beiden dargestellten Ausführungsformen der medizinischen Werkzeuge 1 zeichnen sich dadurch aus, daß der Meißel 2 nicht mehr direkt von der Hand des Operateurs gehalten werden muß, sondern der Meißel 2 an der Handhabe 3 festlegbar ist. Über die Handhabe 3 kann der Operateur den Meißel 2 viel sicherer und zielgenauer führen als dies zuvor möglich war.

### Bezugszeichenliste

- 1: medizinisches Werkzeug
- 2: Meißel
- 2a: Schaft
- 2b: Meißelschneide
- 3: Handhabe
- 4: Handgriff
- 5: Führung
- 6: Schlitz
- 7: Anschlag
- 7a: Anschlagfläche
- 7b: Anschlagfläche
- 8: Gewicht
- 8a: Führungsschlitz
- 9: Anlagevorrichtung/Weichteilschutz
- 9a: Einschlagdom
- 9b: Hammerplattform
- 10: Pfeil

## Patentansprüche

1. Medizinisches Werkzeug zum Durchtrennen, Abtragen und insbesondere Modellieren von Knochen, Knorpeln und dergleichen, insbesondere Nasenrückenosteotom, umfassend einen Meißel (2) mit einem Schaft (2a), einer am distalen Ende des Schaftes (2a) ausgebildeten Meißelschneide (2b), und einer Handhabe, wobei der Meißel (2) zum Halten und Führen an der Handhabe (3) festlegbar ist,
**dadurch gekennzeichnet**
**daß** die Handhabe (3) einen Handgriff (4) sowie eine Führung (5) zur Aufnahme des Meißels (2) umfaßt, wobei der Handgriff (4) und die Führung (5) räumlich voneinander beabstandet sind.

2. Medizinisches Werkzeug nach Anspruch 1, **dadurch gekennzeichnet, daß** die Führung (5) als in einem Abschnitt der Handhabe (3) angeordneter Schlitz (6) ausgebildet ist, wobei die Handhabe (3) in diesem Abschnitt den Querschnitt eines Abschnitts des Meißels (2) zumindest teilweise umgreift, so daß der Meißels (2) kippstabil in dem Schlitz (6) geführt ist.

3. Medizinisches Werkzeug nach Anspruch 1, **dadurch gekennzeichnet, daß** die Führung (5) als an der Handhabe (3) angeordnete Führungsvorrichtung ausgebildet ist, die den Querschnitt eines Abschnitts des Meißels (2) zumindest teilweise umgreift, so daß der Meißel (2) in der Führungsvorrichtung geführt ist.

4. Medizinisches Werkzeug nach mindestens einem der Ansprüche 1 bis 3, **dadurch gekennzeichnet, daß** zumindest die Teile des Schaftes (2a) des Meißels (2) und/oder der Führung (5), die die Gleitpaarung zum Verschieben des Meißels (2) bilden, aus Materialien mit günstigen Gleiteigenschaften, insbesondere einem geringen Reibungskoeffizienten, bestehen.

5. Medizinisches Werkzeug nach mindestens einem der Ansprüche 1 bis 4, **dadurch gekennzeichnet, daß** die Einstecktiefe des Meißels (2) in die Führung (5) einem Vielfachen der Höhe des Querschnitts des Meißels (2) entspricht.

6. Medizinisches Werkzeug nach mindestens einem der Ansprüche 1 bis 5, **dadurch gekennzeichnet, daß** die Führung (5) im Längs- und Querschnitt der Längs- und Querschnittsform des von der Führung (5) aufgenommemen Abschnitts des Meißels (2) entspricht.

7. Medizinisches Werkzeug nach mindestens einem der Ansprüche 1 bis 6, **dadurch gekennzeichnet, daß** an der Führung (5) ein die Einschlaglänge des Meißels (2) begrenzender Anschlag ausgebildet ist.

8. Medizinisches Werkzeug nach mindestens einem der Ansprüche 1 bis 7, **dadurch gekennzeichnet, daß** am Meißel (2) ein die Einschlaglänge des Meißels (2) begrenzender Anschlag (7) ausgebildet ist.

9. Medizinisches Werkzeug nach Anspruch 8, **dadurch gekennzeichnet, daß** der Anschlag (7) als im Bereich der Führung (5) gegen die Handhabe (3) oder die Führingsvorrichtung anlaufende Vergrößerung des Querschnitts des Schaftes (2a) des Meißels (2) ausgebildet ist.

10. Medizinisches Werkzeug nach Anspruch 8, **dadurch gekennzeichnet, daß** der Anschlag (7) entlang der Längsachse des Schaftes (2a) des Meißels (2) verstellbar ist.

11. Medizinisches Werkzeug nach mindestens einem der Ansprüche 8 bis 10, **dadurch gekennzeichnet, daß** der Anschlag (7) die Einschlaglänge des Meißels (2) dämpfend begrenzt.

12. Medizinisches Werkzeug nach mindestens einem der Ansprüche 1 bis 11, **dadurch gekennzeichnet, daß** der Meißel (2) über ein auf den Schaft (2a) des Meißels (2) aufschiebbares Gewicht (8) in Arbeitsrichtung antreibbar ist, wobei am Schaft (2a) des Meißels (2) ein Anschlag (7) als Widerlager für das Gewicht (8) ausgebildet ist.

13. Medizinisches Werkzeug nach Anspruch 12, **dadurch gekennzeichnet, daß** zumindest die Teile des Gewichts (8) und/oder des Schaftes (2a) des Meißels (2), die die Gleitpaarung zum Verschieben des Gewichtes (8) enlang dem Meißelschaft (2a) bilden, aus Materialien mit günstigen Gleiteigenschaften, insbesondere einem geringen Reibungskoeffizienten, bestehen.

14. Medizinisches Werkzeug nach Anspruch 12 oder 13, **dadurch gekennzeichnet, daß** die Außenkontur des Gewichts (8), insbesondere mit Grifflächen versehen, ergonomisch optimiert ausgestaltet ist.

15. Medizinisches Werkzeug nach Anspruch 12, **dadurch gekennzeichnet, daß** der das Widerlager für das Gewicht (8) bildende Anschlag (7) auch den die Einschlaglänge des Meißels (2) begrenzenden Anschlag (7) bildet.

16. Medizinisches Werkzeug nach mindestens einem der Ansprüche 1 bis 15, **dadurch gekennzeichnet, daß** am Meißel (2) im Bereich der Meißelschneide (2b) zumindest einseitig, vorzugsweise beidseitig, ein Meißelklingenschutz angeordnet ist.

17. Medizinisches Werkzeug nach Anspruch 16, **dadurch gekennzeichnet, daß** der Meißelklingenschutz einstückig mit einem Weichteilschutz versehen ist.

18. Medizinisches Werkzeug nach mindestens einem der Ansprüche 1 bis 17, **dadurch gekennzeichnet, daß** an der Handhabe (3) ein als Retraktor wirkender Weichteilschutz (9) angeordnet ist.

19. Medizinisches Werkzeug nach mindestens einem der Ansprüche 1 bis 18, **dadurch gekennzeichnet, daß** an der Handhabe (3) eine den Zielpunkt des Meißels (2) markierende Anlagevorrichtung (9) festlegbar ist.

20. Medizinisches Werkzeug nach Anspruch 19, **dadurch gekennzeichnet, daß** die den Zielpunkt des Meißels (2) markierende Anlagevorrichtung (9) auch den als Retraktor wirkenden Weichteilschutz (9) bildet.

21. Medizinisches Werkzeug nach Anspruch 19 oder 20, **dadurch gekennzeichnet, daß** die Anlagevorrichtung (9) einen Einschlagdorn (9a) zum Festlegen am Zielpunkt aufweist.

22. Medizinisches Werkzeug nach Anspruch 21, **dadurch gekennzeichnet, daß** die Anlagevorrichtung (9) eine Hammerplattform (9b) zum Einschlagen des Einschlagdorns (9a) aufweist.

23. Medizinisches Werkzeug nach mindestens einem der Ansprüche 1 bis 22, **dadurch gekennzeichnet, daß** zumindest alle direkt mit dem Patientenkörper, insbesondere Knochen- und/oder Knorpelteilen, in Kontakt kommenden Bauteile aus körperverträglichen Materialien, insbesondere köperverträglichen Stahllegierungen, hergestellt sind.

## Claims

1. Medical instrument for sectioning, removing and in particular modelling bone, cartilage and the like, in particular a nasal bridge osteotome, comprising a chisel (2) with a shaft (2a), a chisel blade (2b) formed at the distal end of the shaft (2a), and a handle, the chisel (2) being secured for holding and guiding on the handle (3), **characterized in that** the handle (3) comprises a grip (4) and also a guide (5) for receiving the chisel (2), the grip (4) and the guide (5) being set a distance apart from one another.

2. Medical instrument according to Claim 1, **characterized in that** the guide (5) is designed as a slit (6) arranged in a portion of the handle (3), the handle (3) in this portion at least partially engaging around the cross section of a portion of the chisel (2), so that the chisel (2) is guided in the slit (6) in a manner secure against tilting.

3. Medical instrument according to Claim 1, **characterized in that** the guide (5) is designed as a guide device which is arranged on the handle (3) and which at least partially engages around the cross section of a portion of the chisel (2), so that the chisel (2) is guided in the guide device.

4. Medical instrument according to at least one of Claims 1 to 3, **characterized in that** at least those parts of the shaft (2a) of the chisel (2) and/or of the guide (5) which form the slide pairing for moving the chisel (2) are made of materials with favourable slide properties, in particular a low coefficient of friction.

5. Medical instrument according to at least one of Claims 1 to 4, **characterized in that** the depth of insertion of the chisel (2) into the guide (5) corresponds to a multiple of the height of the cross section of the chisel (2).

6. Medical instrument according to at least one of Claims 1 to 5, **characterized in that** the guide (5) corresponds in longitudinal section and cross section to the longitudinal section and cross section shape of the portion of the chisel (2) received by the guide (5).

7. Medical instrument according to at least one of Claims 1 to 6, **characterized in that** a stop limiting the impaction length of the chisel (2) is formed on the guide (5).

8. Medical instrument according to at least one of Claims 1 to 7, **characterized in that** a stop (7) limiting the impaction length of the chisel (2) is formed on the chisel (2).

9. Medical instrument according to Claim 8, **characterized in that** the stop (7) is designed as an enlargement of the cross section of the shaft (2a) of the chisel (2) running against the handle (3) or the guide device in the area of the guide (5).

10. Medical instrument according to Claim 8, **characterized in that** the stop (7) is adjustable along the longitudinal axis of the shaft (2a) of the chisel (2).

11. Medical instrument according to at least one of Claims 8 to 10, **characterized in that** the stop (7) limits the impaction length of the chisel (2) with a damping effect.

12. Medical instrument according to at least one of Claims 1 to 11, **characterized in that** the chisel (2) can be driven in the working direction via a weight (8) that can be pushed onto the shaft (2a) of the chisel (2), a stop (7) as an abutment for the weight (8) being formed on the shaft (2a) of the chisel (2) .

13. Medical instrument according to Claim 12, **characterized in that** at least those parts of the weight (8) and/or of the shaft (2a) of the chisel (2) which form the slide pairing for moving the weight (8) along the chisel shaft (2a) are made of materials with favourable slide properties, in particular a low coefficient of friction.

14. Medical instrument according to Claim 12 or 13, **characterized in that** the outer contour of the weight (8), in particular provided with grip surfaces, has an optimized ergonomic design.

15. Medical instrument according to Claim 12, **characterized in that** the stop (7) forming the abutment for the weight (8) also forms the stop (7) limiting the impaction length of the chisel (2).

16. Medical instrument according to at least one of Claims 1 to 15, **characterized in that** a chisel blade protector is arranged on the chisel (2) in the area of the chisel blade (2b), at least on one side, preferably on both sides.

17. Medical instrument according to Claim 16, **characterized in that** the chisel blade protector is provided integrally with a soft-tissue protector.

18. Medical instrument according to at least one of Claims 1 to 17, **characterized in that** a soft-tissue protector (9) acting as a retractor is arranged on the handle (3).

19. Medical instrument according to at least one of Claims 1 to 18, **characterized in that** a bearing device (9) marking the target point of the chisel (2) can be secured on the handle (3).

20. Medical instrument according to Claim 19, **characterized in that** the bearing device (9) marking the target point of the chisel (2) also forms the soft-tissue protector (9) acting as retractor.

21. Medical instrument according to Claim 19 or 20, **characterized in that** the bearing device (9) has an impaction spike (9a) for fixing at the target point.

22. Medical instrument according to Claim 21, **characterized in that** the bearing device (9) has a hammer platform (9b) for driving home the impaction spike (9a).

23. Medical instrument according to at least one of Claims 1 to 22, **characterized in that** at least all the components coming directly into contact with the patient's body, in particular with bone and/or cartilage parts, are made of biocompatible materials, in particular biocompatible steel alloys.

## Revendications

1. Outil médical pour séparer, déblayer et en particulier modeler des os, des cartilages et similaires, en particulier l'ostéotome du dos du nez, comprenant un burin (2) avec un fût (2a) et un tranchant de burin (2b) réalisé à l'extrémité distale du fût (2a) et une manette, le burin (2) pouvant être immobilisé sur la manette (3) pour le maintien et le guidage, **caractérisé en ce que** la manette (3) comprend une poignée (4) ainsi qu'un guidage (5) pour recevoir le burin (2), la poignée (4) et le guidage (5) étant à distance spatiale l'une de l'autre.

2. Outil médical selon la revendication 1, **caractérisé en ce que** le guidage (5) est réalisé sous forme de fente (6) agencée dans un tronçon de la manette (3), la manette (3) entourant dans ce tronçon au moins partiellement la section transversale d'un tronçon du burin (2) de sorte que le burin (2) est guidé dans la fente (6) de manière stable vis-à-vis du basculement.

3. Outil médical selon la revendication 1, **caractérisé en ce que** le guidage (5) est réalisé sous forme de dispositif de guidage agencé sur la manette (3), lequel entoure au moins partiellement la section transversale d'un tronçon du burin (2), de sorte que le burin (2) est guidé dans le dispositif de guidage.

4. Outil médical selon au moins l'une des revendications 1 à 3, **caractérisé en ce qu'**au moins les parties du fût (2a) du burin (2) et/ou du guidage (5) qui forment la paire d'éléments en coulissement pour déplacer le burin (2), sont constituées par des matériaux à propriétés de coulissement favorables, en particulier avec un faible coefficient de friction.

5. Outil médical selon au moins l'une des revendications 1 à 4, **caractérisé en ce que** la profondeur d'enfoncement du burin (2) dans le guidage (5) correspond à un multiple de la hauteur de la section transversale du burin (2).

6. Outil médical selon au moins l'une des revendications 1 à 5, **caractérisé en ce que** le guidage (5) correspond, dans sa section longitudinale et transversale, à la forme de section longitudinale et transversale du tronçon de burin (2) reçu par le guidage (5).

7. Outil médical selon au moins l'une des revendications 1 à 6, **caractérisé en ce que** sur le guidage (5) est réalisée une butée limitant la longueur de frappe du burin (2).

8. Outil médical selon au moins l'une des revendications 1 à 7, **caractérisé en ce que** sur le burin (2) est réalisé une butée limitant la longueur de frappe du burin (2).

9. Outil médical selon la revendication 8, **caractérisé en ce que** la butée (7) est réalisée sous forme d'agrandissement de la section transversale du fût (2a) du burin (2) venant s'adosser, dans la région du guidage (5), contre la manette (3) ou contre le dispositif de guidage.

10. Outil médical selon la revendication 8, **caractérisé en ce que** la butée (7) est réglable le long de l'axe longitudinal du fût (2a) du burin (2).

11. Outil médical selon au moins l'une des revendications 8 à 10, **caractérisé en ce que** la butée limite avec amortissement la longueur de frappe du burin (2).

12. Outil médical selon au moins l'une des revendications 1 à 11, **caractérisé en ce que** le burin (2) peut être entraîné en direction de travail via un poids (8) qui peut être enfilé sur le fût (2a) du burin (2), une butée (7) servant de contrefort pour le poids (8) étant réalisée sur le fût (2a) du burin (2).

13. Outil médical selon la revendication 12, **caractérisé en ce qu'**au moins les parties du poids (8) et/ou du fût (2a) du burin (2), qui forment la paire d'éléments en coulissement pour déplacer le poids (8) le long du fût (2a) du burin, sont constituées en des matériaux à propriétés de coulissement favorables, en particulier à faible coefficient de friction.

14. Outil médical selon l'une ou l'autre des revendications 12, et 13, **caractérisé en ce que** le contour extérieur du poids (8), pourvu en particulier de surfaces de préhension, est réalisé de manière optimisée du point de vue ergonomique.

15. Outil médical selon la revendication 12, **caractérisé en ce que** la butée (7) formant le contrefort pour le poids (8) forme aussi la butée (7) limitant la longueur de frappe du burin (2).

16. Outil médical selon au moins l'une des revendications 1 à 15, **caractérisé en ce qu'**une protection de lame de burin est agencée sur le burin (2) dans la région du tranchant (2b) du burin, au moins d'un côté, de préférence des deux côtés.

17. Outil médical selon la revendication 16, **caractérisé en ce que** la protection de lame de burin est pourvue, d'un seul tenant, d'une protection pour les parties molles.

18. Outil médical selon au moins l'une des revendications 1 à 17, **caractérisé en ce que** sur la manette (3) est agencée une protection pour les parties molles (9) agissant à titre de rétracteur.

19. Outil médical selon au moins l'une des revendications 1 à 18, **caractérisé en ce qu'**un dispositif d'appui (9) marquant le point cible du burin (2) peut être immobilisé sur la manette (3).

20. Outil médical selon la revendication 19, **caractérisé en ce que** le dispositif d'appui (9) marquant le point cible du burin (2) forme aussi la protection pour les parties molles (9), agissant à titre de rétracteur.

21. Outil médical selon l'une ou l'autre des revendications 19 et 20, **caractérisé en ce que** le dispositif d'appui (9) présente un mandrin de frappe (9a) pour l'immobilisation au point cible.

22. Outil médical selon la revendication 21, **caractérisé en ce que** le dispositif d'appui (9) présente une plateforme de martelage (9b) pour enfoncer le mandrin de frappe (9a).

23. Outil médical selon au moins l'une des revendications 1 à 22, **caractérisé en ce qu'**au moins tous les composants venant en contact avec le corps du patient, en particulier avec des parties d'os ou de cartilages, sont réalisés en matériaux tolérés par le corps, en particulier en alliages d'acier tolérés par le corps.
